# EUROPEAN PATENT APPLICATION

(11) **EP 3 574 889 A1**
(43) Date of publication of application: **04.12.2019**
(21) Application number: 17894162.1
(22) Date of filing: 25.01.2017
(51) Int. Cl.: A61K 8/44, A61K 8/02, A61K 8/31, A61K 8/362, A61K 8/365, A61K 8/37, A61K 8/49, A61Q 1/06

(54) **TRANSPARENT SOLID COSMETIC**

(71) Applicant: Kokyu Alcohol Kogyo Co., Ltd., Narita-shi, Chiba 287-0225 (JP)
(72) Inventor: ARAHIRA, Nana, Narita-shi Chiba 287-0225 (JP); KAWAI, Kiyotaka, Narita-shi Chiba 287-0225 (JP)
(74) Representative: Cabinet Nuss
(86) International application number: PCT/JP2017/002534
(87) International publication number: WO 2018/138802

(57) **Abstract**

The purpose of the present invention is to provide a transparent solid cosmetic that colors when applied to the skin. A transparent solid cosmetic that changes color after application, wherein the transparent solid cosmetic includes two or more amino-acid-type gelling agents including dibutyl lauroyl glutamide (GP-1) and dibutyl hexanoyl glutamide (EB-21), and has a transmittance of 50% or higher.

## Description

### [Technical Field]

The present invention relates to a transparent solid cosmetic which develops color when it is applied on the skin.

### [Background Art]

Conventionally, in the field of makeup cosmetics, colorless or transparent cosmetics which develop color after they are applied on the lips are commercialized.

As a transparent cosmetic which develops color after it is applied on the lips, "Your Lip Only Gloss (a liquid gloss consisting of the following ingredients: diisostearyl malate, hydrogenated polyisobutene, triethylhexanoin, ethylhexyl methoxycinnamate, silica dimethyl silylate, t-butyl methoxydibenzoylmethane, octyldodecanol, dibutyl lauroyl glutamide, ethylparaben, dibutyl ethylhexanoyl glutamide, ethylparaben, tocopherol, polyglyceryl-2 triisostearate, Red 218 (C.I. No . 45410)" and the like are commercialized. However, this is not a transparent solid cosmetic.

Further, as a lip balm which develops color after it is applied on the lips, "Your Lip Only Balm (a solid cosmetic consisting of the following ingredients: diphenyl dimethicone, octyldodecanol, triethylhexanoin, tricyclodecanemethyl isononanoate, dextrin palmitate, ethylhexyl methoxycinnamate, polyglyceryl-2 triisostearate, dibutyl lauroyl glutamide, dibutyl ethylhexanoyl glutamide, t-butyl methoxydibenzoylmethane, phenoxyethanol, tocopherol, Red 218(C.I. No. 45410), Red 104, Blue 1)" and the like are commercialized. However, its degree of transparency is very low, its hardness is high, and there are also problems in adhesiveness to the skin and feeling of use.

Furthermore, a transparent solid cosmetic which develops color after it is applied on the lips, a lip balm which is a Chinese product (Manufacture name: Kailijumei) is commercialized. However, its color shade is slightly yellowish and not colorless transparent.

### [Summary of the Invention]

### [Problems to Be Solved by the Invention]

In the field of solid cosmetics, cosmetic products having high transmittance and providing high pleasantness for enjoying color change after they are applied to the skin are desired. In such a solid cosmetic, it is an object of the present invention to provide high quality solid cosmetics which can maintain higher transparency and which have good adhesiveness.

### [Means for Solving the Problems]

In order to solve the above-mentioned problems, during diligent researches into ingredients for enhancing the degree of transparency in cosmetics, the present inventors have focused on amino acid based gelling agents, dibutyl lauroyl glutamide (GP-1) and dibutylhexanoyl glutamide (EB-21), and have found that solid cosmetics containing these exhibit high colorless transparency, i.e. the degree of transparency is 50% or higher, and as a result of further studies, the present invention has been completed.

Namely, the present invention relates to the following [1] to [11]:
[1] A transparent solid cosmetic which changes its color after it is applied, wherein it comprises two or more amino acid based gelling agents including dibutyl lauroyl glutamide and dibutyl hexanoyl glutamide, and its transmittance is 50% or higher.
[2] The transparent solid cosmetic according to [1], wherein the concentration of the amino acid based gelling agents is 3 to 8%.
[3] The transparent solid cosmetic according to [1] or [2], wherein the blending ratio of dibutyl lauroyl glutamide (GP-1) and dibutyl hexanoyl glutamide (EB-21) is 75:25 to 40:60.
[4] The transparent solid cosmetic according to any one of [1] to [3], further comprising a water soluble organic acid.
[5] The transparent solid cosmetic according to any one of [1] to [4], wherein the water soluble organic acid is an acid having pH of 3 or less in 0.1% aqueous solution.
[6] The transparent solid cosmetic according to [4] or [5], wherein the water soluble organic acid is oxalic acid, malic acid, citric acid, succinic acid, phosphoric acid or lactic acid.
[7] The transparent solid cosmetic according to any one of [4] to [6], wherein the water soluble organic acid is citric acid or phosphoric acid.
[8] The transparent solid cosmetic according to any one of [1] to [7], further comprising a hydrocarbon oil and an oil agent.
[9] The transparent solid cosmetic according to [8], wherein the blending proportion of the hydrocarbon oil is 25 to 75 % by weight.
[10] The transparent solid cosmetic according to [8] or [9], wherein the hydrocarbon oil is hydrogenated polyisobutene.
[11] The transparent solid cosmetic according to any one of [8] to [10], wherein the oil agent is triethylhexanoin, hexyl laurate, diethylhexyl succinate, isotridecyl isononanoate, neopentyl glycol diethylhexanoate, isobutyl isostearate, cetyl ethylhexanoate, ethylhexyl palmitate, isostearyl neopentanoate, neopentyl alcohol dicaprate, neopentyl glycol diisononanoate, caprylic / capric triglyceride, triethylhexanoin, tricyclodecane methyl isononanoate, trimethylolpropane triethylhexanoate, octyldodecanol, ethylhexyl hydroxystearate, isostearic acid, octyldodecyl stearoyloxystearate, diisopropyl dilinoleate, triisostearin, trimethylolpropane triisostearate, pentaerythrityl tetraisostearate, polyglyceryl-2 triisostearate, polyglyceryl-2 diisostearate, dipentaerythrityl hexaisononanoate, or diisostearyl malate.

### [Advantageous Effects of the Invention]

When looking for materials that exhibit higher degree of transparency as materials for a transparent solid cosmetic that change its color after it is applied, dibutyl lauroyl glutamide (GP-1) and dibutyl hexanoyl glutamide (EB-21) are substances that are difficult to handle when prepared on an industrial scale; although in recent years a blend with octyldodecanol or cetanol (manufactured by Kokyu Alcohol Kogyo Co., Ltd .: AJK-OD 2046 etc.) that makes them easy to handle, has become used, its preparation is still not easy. It is surprising that a colorless solid cosmetic with 50% or higher degree of transparency, which have never made before, can be obtained by venturing to blend dibutyl lauroyl glutamide (GP-1) and dibutyl hexanoyl glutamide (EB-21) which have not been used for this application. Moreover, simultaneously, the present invention has also been able to achieve a solid cosmetic with good adhesiveness and feeling of use.

For the solid cosmetics of the present invention, the gel strength can be adjusted by adjusting the concentration of the gelling agents and the transmittance can be adjusted by adjusting the blending ratio between the amino acid based gelling agents.

Further, in the present invention, when a fluorescein type dye such as the dye with C.I. number of 45410 etc. is especially used as a dye, colorless transparency can be maintained by preventing the lactone moiety of the fluorescein structure of the dye from ring opening to a carboxylic acid; thereby obtaining high quality solid cosmetics which can develop color after it is applied on the skin. Furthermore, in the transparent cosmetics of the present invention, the solid cosmetics with enjoyable appearance can be made by adding various decorations.

### [Brief Description of Drawings]

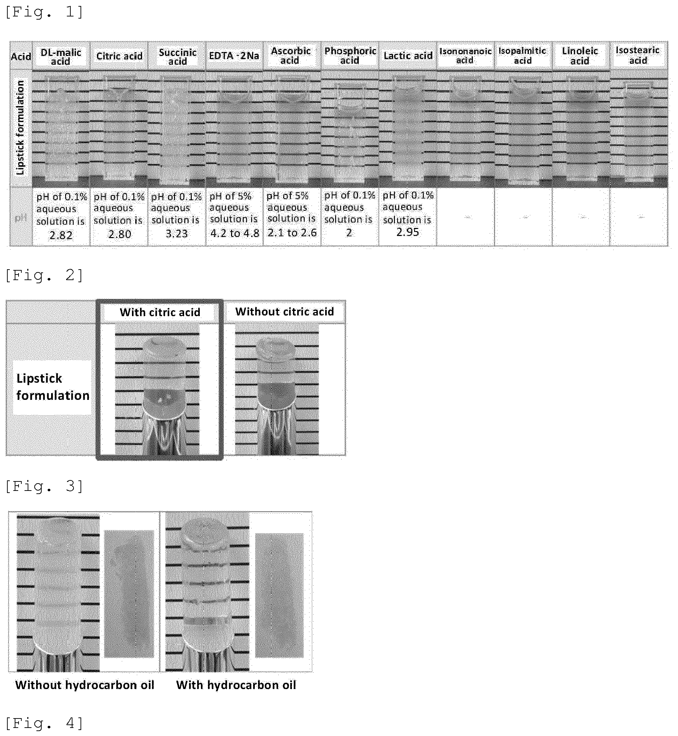

[FIG.1] FIG. 1 is a diagram comparing the effects of the addition of an acid in the formulation of Example 1 using trihexanoin as the oil agent and hydrogenated polyisobutene as the hydrocarbon oil. It was colorless when malic acid, citric acid, succinic acid, phosphoric acid, and lactic acid were used, while it was red when EDTA·2Na, ascorbic acid, isononanoic acid, isopalmitic acid, linoleic acid and isostearic acid were used.
[FIG.2] FIG. 2 is a diagram comparing the difference in the degree of transparency depending on the presence or absence of citric acid in Example 2. It was colorless in the presence of citric acid while it was red in the absence of citric acid.
[FIG. 3] FIG. 3 is a diagram comparing the difference in the degree of transparency depending on the presence or absence of the hydrocarbon oil in Example 3. It was cloudy in the absence of the hydrocarbon oil while it was transparent in the presence of the hydrocarbon oil. Moreover, it turned red after it is applied.
[FIG. 4] FIG. 4 is a diagram comparing the difference in the degree of transparency depending on the blending ratio of triethylhexanoin and hydrogenated polyisobutene in Example 4. It was almost colorless when the blending ratio of triethylhexanoin and hydrogenated polyisobutene was 95:0 to 30:65, while it was red when the ratio was 20:75 to 0:95.

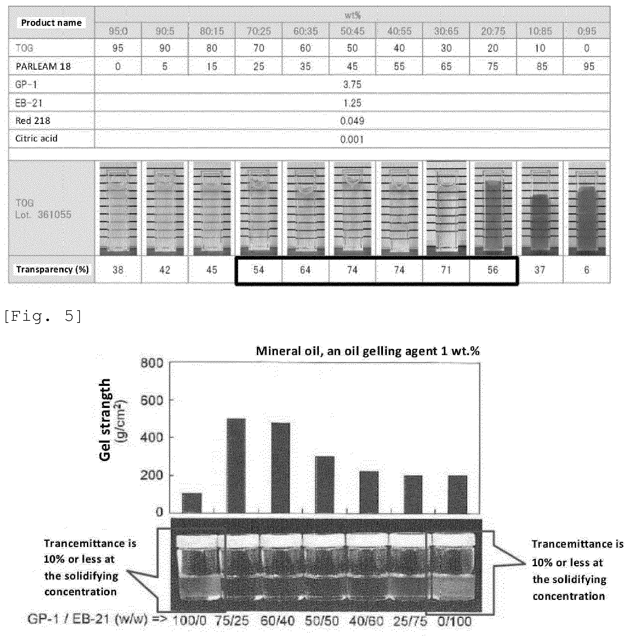

[FIG. 5] FIG. 5 is a diagram comparing the gel strength and the transmittance in Example 5 in which the blending ratio of dibutyl lauroyl glutamide (GP-1) and dibutyl hexanoyl glutamide (EB-21) was changed. It was colorless when GP-1:EB-21 was 75:25 to 40:60, while it was blue when the ratio was 100:0, 25:75 and 0:100.

### [Detailed Description of the Invention]

Oil agents which can be used in the present invention include, but not limited to, triethylhexanoin, hexyl laurate, diethylhexyl succinate, isotridecyl isononanoate, neopentyl glycol diethylhexanoate, isobutyl isostearate, cetyl ethylhexanoate, ethylhexyl palmitate, isostearyl neopentanoate, neopentyl alcohol dicaprate, neopentyl glycol diisononanoate, caprylic / capric triglyceride, triethylhexanoin, tricyclodecanemethyl isononanoate, trimethylolpropane triethylhexanoate, octyldodecanol, ethylhexyl hydroxystearate, isostearic acid, octyldodecyl stearoyloxystearate, diisopropyl dilinoleate, triisostearin, trimethylolpropane triisostearate, pentaerythrityl tetraisostearate, polyglyceryl-2 triisostearate, polyglyceryl-2 diisostearate, dipentaerythrityl hexaisononanoate, diisostearyl malate,
diisobutyl adipate, hexyldecyl ethylhexanoate, ethylhexyl isononanoate, isodecyl isononanoate, isotridecyl isononanoate, isopropyl myristate, isopropyl palmitate, isopropyl isostearate, dipentaerythrityl hexahydroxystearate / hexastearate / hexarosinate, hydrogenated castor oil isostearate, hydrogenated castor oil dimer dilinoleate, polyglyceryl-2 isostearate / dimer dilinoleate copolymer, polyglyceryl-2 diisostearate / dimer dilinoleate copolymer, phytosteryl / isostearyl / cetyl / stearyl / behenyl dimer dilinoleate, bis-phytosteryl / behenyl / isostearyl dimer dilinoleyl dimer dilinoleate, phytosteryl isostearyl dimer dilinoleate, dimer dilinoleyl hydrogenated rosinate, dimer dilinoleyl diisostearate, dimer dilinoleyl dimer dilinoleate, cholesteryl / behenyl / octyldodecyl lauroyl glutamate, phytosteryl / behenyl / octyldodecyl lauroyl glutamate, phytosteryl / decyltetradecyl myristoyl methyl alaninate, diglycerin / dilinoleic acid / hydroxystearic acid copolymer etc.;

Preferably, triethylhexanoin, hexyl laurate, diethylhexyl succinate, isotridecyl isononanoate, neopentyl glycol diethylhexanoate, isobutyl isostearate, cetyl ethylhexanoate, ethylhexyl palmitate, isostearyl neopentanoate, neopentyl alcohol dicaprate, neopentyl glycol diisononanoate, caprylic / capric triglyceride, triethylhexanoin, tricyclodecanemethyl isononanoate, trimethylolpropane triethylhexanoate, octyldodecanol, ethylhexyl hydroxystearate, isostearic acid, octyldodecyl stearoyloxystearate, diisopropyl dilinoleate, triisostearin, trimethylolpropane triisostearate, pentaerythrityl tetraisostearate, polyglyceryl-2 triisostearate, polyglyceryl-2 diisostearate, dipentaerythrityl hexaisononanoate, diisostearyl malate can be used.

Dye which can be used in the present invention include, but not limited to, dyes with the following C.I. numbers: C.I.20170, C.I.20470, C.I.60725, C.I.60730, C.I.42090, C.I.73015, C.I.73000, C.I.42052, C.I.69825, C.I.42090, C.I.61520, C.I.74160, C.I.61570, C.I.61565, C.I.59040, C.I.42095, C.I.42053, C.I.10020, C.I.42085, C.I.45350, C.I.47005, C.I.47000, C.I.21090, C.I.19140, C.I.11680, C.I.18950, C.I.10316, C.I.11380, C.I.11390, C.I.13065, C.I.18820, C.I.15985, C.I.45370, C.I.12075, C.I.21110, C.I.15510, C.I.45425, C.I.11725, C.I.14600, C.I.12100, C.I.16255, C.I.45410, C.I.45440, C.I.45100, C.I.16185, C.I.15850, C.I.15585, C.I.15630, C.I.45170, C.I.15800, C.I.15880, C.I.12120, C.I.45380, C.I.26100, C.I.73360, C.I.17200, C.I.12085, C.I.45430, C.I.45190, C.I.12315, C.I.15865, C.I.26105, C.I.16155, C.I.16150, C.I.14700, C.I.12140, C.I.15620; preferably fluorescein type dyes with the following C. I. numbers: C.I.45350, C.I.45370, C.I.45425, C.I.45410, C.I.45440, C.I.45100, C.I.45170, C.I.45380, C.I.45430, C.I.45190; and most preferably a dye with C.I. number of C.I.45410.

The water soluble organic acid used together with the dye in the present invention is an acid having a pH of 3 or less in 0.1% aqueous solution, and is, but not limited to, for example, oxalic anhydride, oxalic acid, malic acid, citric acid, succinic acid, phosphoric acid, lactic acid, glycolic acid, tartaric acid, etc.; preferably malic acid, citric acid, succinic acid, phosphoric acid or lactic acid; more preferably malic acid, citric acid, succinic acid; particularly preferably citric acid or phosphoric acid.

Further, the blending amount of the water soluble organic acid is 0.0001% to 5.0%, preferably 0.001% to 0.1%.

In the present invention, a hydrocarbon oil is used to increase the degree of transparency. The hydrocarbon oil is not limited but hydrogenated polyisobutene can be used; and for example, depending on its degree of polymerization, PARLEAM 18 or PARLEAM 24 can be used, but it is not limited thereto.

The concentration of the amino acid based gelling agent is determined in view of gel strength, adhesiveness to the skin, feeling of use and the like, and its concentration is preferably 3 to 8%.

The blending ratio between the amino acid based gelling agents is determined in view of transmittance, and its bending ratio of GP-1: EB-21 is preferably in the range of 75:25 to 40: 60%.

### EXAMPLE 1.

The effects of the addition of various acids were compared in the following formulations using trihexanoin as the oil agent and hydrogenated polyisobutene as the hydrocarbon oil.

**Table 1.**

| | Ingredient name | Wt. % |
|---|---|---|
| 1 | Triethylhexanoin | 49.95 |
| 2 | Hydrogenated polyisobutene | 40.0 |
| 3 | Various acids | 5.0 |
| 4 | Dibutyl lauroyl glutamide | 3.75 |
| 5 | Dibutyl hexanoyl glutamide | 1.25 |
| 6 | Red 218 | 0.05 |

Results are shown in Fig. 1.

It became transparent when malic acid, citric acid, succinic acid, phosphoric acid or lactic acid was used. From the above results, it was found that it is transparent when the acid used is a water soluble acid having a pH of 3 or less in 0.1% aqueous solution.

### EXAMPLE 2

Lipsticks were manufactured, and the difference in the degree of transparency due to the presence or absence of citric acid when using the lot of the hydrocarbon oil not producing color and the lot of the hydrocarbon oil producing color was compared.

**Table 2.**

| | Ingredient name | Wt. % |
|---|---|---|
| 1 | Triethylhexanoin | 59.95 |
| 2 | Hydrogenated polyisobutene | 30.00 |
| 3 | Isostearic acid EX | 6.00 |
| 4 | Dibutyl lauroyl glutamide | 3.00 |
| 5 | Dibutyl hexanoyl glutamide | 1.00 |
| 6 | Red 218 | 0.05 |
| 7 | Citric Acid | 0.001 |

Results are shown in Fig. 2.

By using citric acid it was possible to produce transparent lipsticks. In addition, the obtained lipsticks were excellent in gloss and had a smooth feeling of use.

### EXAMPLE 3

A lipsticks were manufactured according to the following formulation comprising isostearic acid, and the degree of transparency thereof was compared with that of a lipstick not comprising a hydrocarbon oil or/and isostearic acid.

**Table 3**

| | Ingredient name | Wt. % | | |
|---|---|---|---|---|
| | | Without hydrocarbon oil and Isostearic acid | Without Isostearic acid | With Isostearic acid |
| 1 | Triethylhexanoin | 94.45 | 49.95 | 49.95 |
| 2 | Hydrogenated polyisobutene | - | 45.0 | 40.0 |
| 3 | Isostearic acid | - | - | 5.0 |
| 4 | Dibutyl lauroyl glutamide | 3.75 | | |
| 5 | Dibutyl hexanoyl glutamide | 1.25 | | |
| 6 | Red 218 | 0.05 | | |

Results are shown in Fig. 3.

The lipstick became cloudy in the absence of hydrogenated isobutene which is hydrocarbon oil. Moreover, although preparation thereof was possible even if it did not contain isostearic acid, when it contained isostearic acid, it turned out that the solubility of dibutyl lauroyl glutamide and dibutyl hexanoyl glutamide increases and the degree of transparency increases.

### EXAMPLE 4

The difference in the degree of transparency depending on the blending ratio of oil agents (triethylhexanoin (TOG)) and isobutene was compared.

The results are shown in Fig. 4.

It was found that the transmittance was 50% or higher when the content of hydrogenated polyisobutene was 25 to 75% by weight.

### EXAMPLE 5

Changes in the gel strength and the transmittance due to differences in the blending ratio of dibutyl lauroyl glutamide (GP-1) and dibutyl hexanoyl glutamide (EB-21) were examined.

**TABLE 4. The gel strength when gelling agents (GP-1:EB-21) = 75:25**

| Gel concentration | Gel strength |
|---|---|
| 3% | Not solidified depending on the oil agent used |
| 4% | Almost Solidified |
| 5% | Almost Solidified |
| 6% | Solidified |

Results are shown in Fig. 5.

It was confirmed that dibutyl lauroyl glutamide (GP-1) and dibutyl hexanoyl glutamide (EB-21) each gave extremely low transmittance when used alone, while when the blending ratio of dibutyl lauroyl glutamide : dibutyl hexanoyl glutamide is 75 to 40% : 25 to 60%, high transmittance was obtained.

## Claims

1. A transparent solid cosmetic which changes its color after it is applied, wherein it comprises two or more amino acid based gelling agents including dibutyl lauroyl glutamide (GP-1) and dibutyl hexanoyl glutamide(EB-21), and its transmittance is 50% or higher.

2. The transparent solid cosmetic according to Claim 1, wherein the concentration of the amino acid based gelling agents is 3 to 8 %.

3. The transparent solid cosmetic according to Claim 1 or 2, wherein the blending ratio of dibutyl lauroyl glutamide (GP-1) and dibutyl hexanoyl glutamide(EB-21) is 75:25 to 40:60.

4. The transparent solid cosmetic according to any one of Claims 1 to 3, further comprising a water soluble organic acid.

5. The transparent solid cosmetic according to any one of Claims 1 to 4, wherein the water soluble organic acid is an acid having pH of 3 or less in 0.1% aqueous solution.

6. The transparent solid cosmetic according to Claim 4 or 5, wherein the water soluble organic acid is oxalic acid, malic acid, citric acid, succinic acid, phosphoric acid or lactic acid.

7. The transparent solid cosmetic according to any one of Claims 4 to 6, wherein the water soluble organic acid is citric acid or phosphoric acid.

8. The transparent solid cosmetic according to any one of Claims 1 to 7, further comprising a hydrocarbon oil and an oil agent.

9. The transparent solid cosmetic according to Claim 8, wherein the blending proportion of the hydrocarbon oil is 25 to 75 % by weight.

10. The transparent solid cosmetic according to Claim 8 or 9, wherein the hydrocarbon oil is hydrogenated polyisobutene.

11. The transparent solid cosmetic according to any one of Claims 8 to 10, wherein the oil agent is triethylhexanoin, hexyl laurate, diethylhexyl succinate, isotridecyl isononanoate, neopentyl glycol diethylhexanoate, isobutyl isostearate, cetyl ethylhexanoate, ethylhexyl palmitate, isostearyl neopentanoate, neopentyl alcohol dicaprate, neopentyl glycol diisononanoate, caprylic / capric triglyceride, triethylhexanoin, tricyclodecanemethyl isononanoate, trimethylolpropane triethylhexanoate, octyldodecanol, ethylhexyl hydroxystearate, isostearic acid, octyldodecyl stearoyloxystearate, diisopropyl dilinoleate, triisostearin, trimethylolpropane triisostearate, pentaerythrityl tetraisostearate, polyglyceryl-2 triisostearate, polyglyceryl-2 diisostearate, dipentaerythrityl hexaisononanoate, or diisostearyl malate.
